(19) Europäisches Patentamt / European Patent Office / Office européen des brevets

(11) **EP 3 682 751 A1**

(12) **EUROPEAN PATENT APPLICATION**
published in accordance with Art. 153(4) EPC

(43) Date of publication:
22.07.2020 Bulletin 2020/30

(51) Int Cl.:
*A24F 47/00* (2020.01)

(21) Application number: 17924923.0

(22) Date of filing: 27.12.2017

(86) International application number:
PCT/CN2017/118838

(87) International publication number:
WO 2019/052082 (21.03.2019 Gazette 2019/12)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD TN

(30) Priority: 12.09.2017 CN 201710818181

(71) Applicant: Changzhou Patent Electronic Technology Co., Ltd
Changzhou, Jiangsu 213001 (CN)

(72) Inventors:
• QIU, Weihua
  Changzhou
  Jiangsu 213164 (CN)
• WANG, Zhongzhou
  Changzhou
  Jiangsu 213164 (CN)

(74) Representative: Zaboliene, Reda
METIDA Law Firm Zaboliene and Partners
Business center VERTAS
Gyneju 16
01109 Vilnius (LT)

(54) **METHOD FOR OBTAINING INTAKE QUANTITY OF TARGET SUBSTANCE AND ELECTRONIC DEVICE**

(57) A method for obtaining the intake quantity of a target substance and an electronic device. The method comprises: obtaining consumption information of a carrier within a statistical period, the consumption information comprising at least one of a first consumption quantity of the carrier within the statistical period, a second consumption quantity of the carrier within a valid time period during a single intake, and a third consumption quantity of the carrier within each unit time period in the valid time period; and obtaining the intake quantity of a target substance within the statistical period according to the consumption information and the content of the target substance in the carrier. The present invention has a reference effect during smoking quitting of a user by using an electronic cigarette, and can help the user actively stop smoking.

obtaining consumption information of a carrier within a statistical period, the consumption information includes at least one of a first consumption quantity of the carrier within the statistical period, a second consumption quantity of the carrier in the valid time period in a single intake process, and a third consumption of the carrier per unit time period within the statistical period — 110

obtaining an intake quantity of the target substance within the statistical period according to the consumption information of the carrier and content of the target substance in the carrier — 120

**FIG.1**

EP 3 682 751 A1

## Description

This application claims priority to Chinese Patent Application No. 200910818181.2, entitled "method for obtaining nicotine inhalation and electronic cigarette", filed on September 12, 2017, the entire contents of which are incorporated herein by reference in the application.

## FIELD OF THE INVENTION

The present invention relates to the field of computer technology, and in particular, to a method and an electronic device for obtaining an intake quantity of a target substance.

## BACKGROUND OF THE INVENTION

At present, as an alternative to tobacco products, e-cigarettes are increasingly popular in the market because of their portability and large quantity of smoke. In general, when an user smokes an electronic cigarette, an air flow is generated which is detected by the sensor, thereby triggering the atomizing assembly to atomize the liquid fluids. Liquid fluids include propylene glycol, nicotine, etc.

Among them, nicotine is a harmful substance that will cause dependence on the smoker. Repeated consumption of nicotine will increase the heart rate and blood pressure of the smoker. Large doses of nicotine can also cause vomiting and nausea. In severe cases, it may even harm the use of food. The life of the person.

## SUMMARY OF THE INVENTION

In order to solve the problem in the prior art that the user ingests too many the target substance in the process of using the electronic cigarette, thereby injuring the health of the body, the present invention provides a method and an electronic device for obtaining the intake quantity of the target substance. The technical solution is as follows:

The present invention provides a method for obtaining an intake quantity of a target substance, the method including: obtaining consumption information of a carrier within a statistical period, the consumption information including at least one of a first consumption quantity of the carrier within the statistical period, a second consumption quantity of the carrier in a valid time period in a single intake process, and a third consumption quantity of the carrier in each unit time period of the valid time period; obtaining the intake quantity of the target substance within the statistical period according to the consumption information and content of the target substance in the carrier.

In an embodiment, the obtaining the consumption information of a carrier within a statistical period includes: using a liquid level detector to detect a first change quantity of liquid level within the statistical period, and determining the first consumption quantity according to the first change quantity; or, using the liquid level detector to detect a second change quantity of the liquid level in the valid time period, and determining the second consumption quantity according to the second change quantity.

In an embodiment, the obtaining the consumption information of a carrier within a statistical period includes: obtaining a working mode of an electronic cigarette in each valid period of time within the statistical period; obtaining the consumption information of the carrier within the statistical period according to the working mode of each valid time period.

In an embodiment, the obtaining the consumption information of the carrier within the statistical period according to the working mode of each valid time period includes: if the electronic cigarette is in a temperature control mode during the valid time period, calculating the third consumption quantity of the carrier in the unit time period according to the temperature of an atomizing assembly in the unit time period every unit time period; or, if the electronic cigarette is in the temperature control mode during the valid time period, obtaining an average temperature of the atomizing assembly in the valid time period; and calculating the second consumption of the carrier during the valid time period according to the average temperature of the atomizing assembly in the valid time period.

In an embodiment, the obtaining the consumption information of the carrier within the statistical period according to the working mode of each valid time period includes: if the electronic cigarette is in a variable waits mode or a bypass mode during the valid time period, calculating the third consumption quantity of the carrier in the unit time period according to output power of the electronic cigarette in the unit time period every unit time period; or, obtaining an average output power of the electronic cigarette in the valid time period if the electronic cigarette is in the variable walts mode or the bypass mode during the valid time period; and calculating the second consumption quantity of the carrier during the valid time period according to the average output power of the electronic cigarette in the valid time period.

In an embodiment, after obtaining the intake quantity of the target substance within the statistical period, the method further includes: controlling the electronic device to perform a preset operation according to the intake quantity.

In an embodiment, controlling the electronic device to perform a preset operation according to the intake quantity includes: detecting whether the intake quantity reaches a first threshold corresponding to the statistical period; when the intake quantity reaches the first threshold, reducing the output power of the atomizing assembly, and/or presenting prompt information for prompting an user that the intake quantity is about to exceed the limit.

In an embodiment, controlling the electronic device to perform a preset operation according to the intake quantity includes: determining a limit intake quantity of a next statistical period according to the intake quantity of the target substance during the statistical period, at the end of the statistical period.

In an embodiment, after obtaining the intake quantity of the target substance within the statistical period, the method further includes: displaying prompt information according to the intake quantity, and the prompt information is used to prompt the intake quantity of the target substance.

In an embodiment, displaying prompt information according to the intake quantity includes: determining a preset pattern corresponding to the intake quantity; prompting the intake quantity by displaying the preset pattern; or displaying the intake quantity of the target substance within the statistical period.

In an embodiment, after obtaining the intake quantity of the target substance within the statistical period, the method further includes: obtaining a first limit intake quantity of the target substance corresponding to the statistical period; calculating a ratio of the intake quantity to the first limit intake quantity; displaying the ratio, or displaying the ratio by displaying a progress bar.

In an embodiment, the valid time period is a time when an airflow sensor starts detecting the airflow to a time when the airflow sensor detects the end of the airflow.

In an embodiment, the valid time period is a time when a cigarette lighter button is turned on to a time when the cigarette lighter button is turned off.

In an embodiment, if a first time is earlier than a second time, start time of the valid time period is the second time; and/or,

if the first time is later than the second time, and time interval between the first time and the second time is less than a second threshold, the start time of the valid time period is the second time; and/or,

if the first time is later than the second time, and the time interval between the first time and the second time is greater than the second threshold, the start time of the valid time period is the first time;

and/or, the cut-off time of the valid time period is an earlier one of the third time and the fourth time;

wherein, the first time is the time when a suction signal is received, the second time is the time when the cigarette lighter button is turned on, and the third time is the time when a cigarette lighter button is turned off, the fourth time is the time when the suction signal is not received.

The present invention also provides an electronic device including a memory and a processor; the memory is configured to storing executable program code; and the processor is configured to invoke the executable program code in the memory to execute the method of obtaining an intake quantity of target substance.

The beneficial effects brought by the technical solutions provided by the embodiments of the present invention are:

The invention obtains the consumption information of the carrier within the statistical period, the consumption information includes at least one of the first consumption quantity of the carrier within the statistical period, the second consumption quantity of the carrier in the valid time period in a single intake process, and the third consumption quantity of the carrier per unit time period of the valid time period; and the intake quantity of the target substance within the statistical period is obtained according to the consumption information of the carrier and the content of the target substance in the carrier. The user can know the intake quantity of the target substance within the statistical period, thereby helping the user to control the intake quantity of the target substance, and solving the problem of the related art in which the user ingests too many target substance in the process of using the electronic cigarette, thereby jeopardizing the health of the body. It plays a reference role in the process of users using e-cigarettes to quit smoking, which can help users to stop smoking actively.

The above description is only an overview of the technical solutions of the present invention, and the above-described and other objects, features and advantages of the present invention can be more clearly understood. Hereinafter, preferred embodiments will be described in detail with reference to the accompanying drawings.

## DRAWINGS

In order to more clearly illustrate the technical solutions in the embodiments of the present invention, the drawings used in the description of the embodiments will be briefly described below. It is obvious that the drawings in the following description are only some embodiments of the present invention. Other drawings may also be obtained from those of ordinary skill in the art in light of the inventive work.

FIG. 1 is a flow chart of a method for obtaining an intake quantity of a target substance provided in an embodiment of the present invention;

FIG.2 is a flow chart of a method for obtaining an intake quantity of a target substance provided in another embodiment of the present invention;

FIG.3-1 is a schematic view showing the position of a liquid level detector in a liquid storage member provided in another embodiment of the present invention;

FIG.3-2 is a flow chart of a method for obtaining an intake quantity of a target substance provided in still another embodiment of the present invention;

FIG.4 is a flow chart of a method for obtaining an intake quantity of a target substance provided in still another embodiment of the present invention;

FIG. 5 is a flow chart showing the manner in which the preset pattern is presented to prompt the intake quantity of the target substance within the statistical period provided in another embodiment of the present invention;

FIG. 6 is a schematic block diagram of an electronic device provided in an embodiment of the present invention.

## DETAILED DESCRIPTION OF PREFERRED EMBODIMENTS

In order to further explain the technical means and efficacy of the present invention for achieving the intended purpose of the present invention, the method for obtaining the intake quantity of the target substance according to the present invention and its specific implementation manner, together with the accompanying drawings and preferred embodiments, structure, characteristics and efficacy, as detailed below.

The above and other technical contents, features and advantages of the present invention will be apparent from the following detailed description of the preferred embodiments of the invention. The technical means and functions of the present invention for achieving the intended purpose can be more deeply and specifically understood by the description of the specific embodiments. However, the drawings are only for the purpose of reference and description, and are not intended to limit.

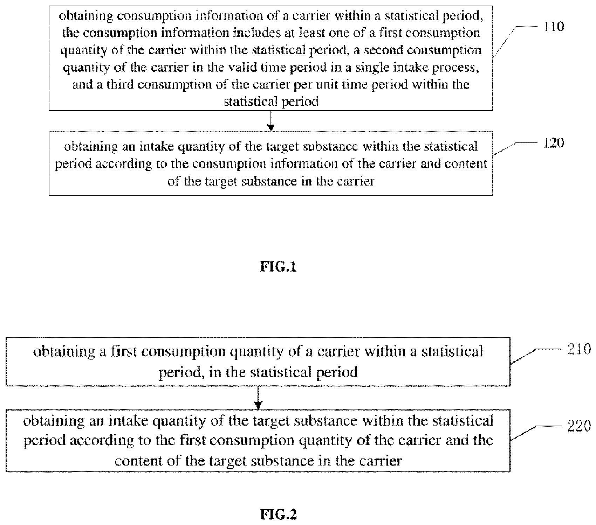

Please refer to FIG. 1, which shows a flow chart of a method for obtaining an intake quantity of a target substance provided in an embodiment of the present invention. As shown in FIG. 1, the method for obtaining the intake quantity of the target substance may include:

Step 110: obtaining consumption information of a carrier within a statistical period, the consumption information includes at least one of a first consumption quantity of the carrier within the statistical period, a second consumption quantity of the carrier in the valid time period in a single intake process, and a third consumption of the carrier per unit time period within the statistical period.

Step 120: obtaining an intake quantity of the target substance within the statistical period according to the consumption information of the carrier and content of the target substance in the carrier.

In summary, the method provided by the embodiment of the present invention obtains the consumption information of the carrier within the statistical period, and the consumption information includes at least one of the first consumption quantity of the carrier within the statistical period, the second consumption quantity of the carrier in a valid time period in a single intake process and a third consumption quantity of the carrier in each unit time period in the valid time period; obtaining the intake quantity of the target substance within the statistical period according to the consumption information and the content of the target substance in the carrier. The user can know the intake quantity of the target substance within the statistical period, thereby helping the user to control the intake quantity of the target substance, and solving problem in the related art that the user ingests too many the target substance in the process of using the electronic cigarette, thereby jeopardizing the health of the body. It plays a reference role in the process of users using e-cigarettes to quit smoking, which can help users to stop smoking actively.

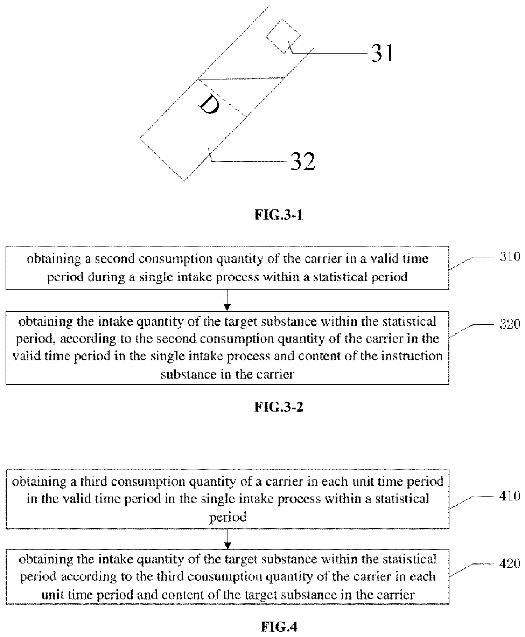

The specific embodiments for obtaining the intake quantity of the target substance in the present application are respectively described in the following three embodiments. For details, refer to the embodiment corresponding to FIG. 2, the embodiment corresponding to FIG. 3-2, and the embodiment corresponding to FIG. 4.

Please refer to FIG. 2, which shows a flow chart of a method for obtaining an intake quantity of a target substance provided in another embodiment of the present invention. As shown in FIG. 2, the method for obtaining the intake quantity of the target substance may include:

Step 210: obtaining a first consumption quantity of a carrier within a statistical period, in the statistical period.

Specifically, the statistical period involved in the present application may be a value set by an user, or may be a default fixed value stored by an electronic device, and the statistical period may be, for example, one day, one week, or one month, and the like.

The carrier involved in the present application may be a carrier such as a liquid smoke, a medical liquid, a sesame paste or the like, and the target substance in the carrier may be a substance in the carrier which can endanger human health. For example, when the carrier is a liquid smoke, the target substance in the carrier involved in the present application may be nicotine; for example, when the carrier is a sesame paste, the target substance in the carrier involved in the present application may include at least one of ephedrine and phleophenol. For example, when the carrier is an anesthetic, the target substance in the carrier involved in the present application may include at least one of acetaminophen, acetyl alpha fentanyl, and acesulfame.

In the case where the carrier in step 210 is a liquid, the electronic device includes a liquid storage member for storing the liquid, and the liquid storage member provides with a liquid level detector for detecting the liquid level of the liquid in the liquid storage member. The step 210 may be implemented by: using a liquid level detector to detect a first change quantity of the liquid level within the statistical period, and determining a first consumption quantity of the liquid in the statistical period according to the first change quantity.

Specifically, the first liquid level detected by the liquid level detector is acquired at the beginning of the statistical period, and the first posture of the liquid storage member is acquired; any time within the statistical period, such as the end of the statistical period, obtaining a second liquid level detected by the liquid level detector, and obtaining a second posture of the liquid storage member; calculating a first liquid quantity at the starting time according to the first liquid level and the first posture, calculating the second liquid quantity at any one time according to the second liquid level and the second posture; the first quantity of liquid within the statistical period is obtained by subtracting the second quantity of liquid from the first quantity of liquid.

Wherein, since the position of the liquid storage member in the electronic device is generally fixed, the posture of obtaining the liquid storage member may be realized: the electronic device is further provided with a gyroscope, and the posture of the electronic device is determined according to the information collected by the gyroscope in the electronic device. Determining the posture of the liquid storage member according to the posture of the electronic device.

Taking the liquid level detector as a laser type sensor and the liquid storage member as a cylinder, as shown in FIG. 3-1, the laser type sensor 31 is installed at the center of the top of the liquid storage member 32. If it is determined that the posture of the liquid storage member is inclined by 45°, the diameter of the liquid storage member is D cm, and the liquid level value detected by the liquid level detector is h cm, the volume of the liquid in the current liquid storage member can be calculated as $\frac{\pi D^3}{2} + \left(h - \frac{D}{2}\right)\pi D^2$

Step 220: obtaining an intake quantity of the target substance within the statistical period according to the first consumption quantity of the carrier and the content of the target substance in the carrier.

Wherein, the content of the target substance in the carrier can be stored in the electronic device by the developer setting, or can be set by an user.

The carrier is used as a liquid smoke for illustration. Assuming that the first consumption quantity of tobacco liquid is 30 ml within a statistical period and the content of nicotine in the liquid is 0.2 mg/ml, the intake quantity of nicotine in this statistical period is 6 mg.

In summary, the method provided by the embodiment of the present invention obtains the consumption information of the carrier within the statistical period, and the consumption information includes at least one of the first consumption quantity of the carrier within the statistical period, the second consumption quantity of the carrier in a valid time period in a single intake process and a third consumption quantity of the carrier in each unit time period in the valid time period; obtaining the intake quantity of the target substance within the statistical period according to the consumption information and the content of the target substance in the carrier. The user can know the intake quantity of the target substance within the statistical period, thereby helping the user to control the intake quantity of the target substance, and solving problem in the related art that the user ingests too many the target substance in the process of using the electronic cigarette, thereby jeopardizing the health of the body. It plays a reference role in the process of users using e-cigarettes to quit smoking, which can help users to stop smoking actively.

Please refer to FIG. 3-2, which shows a flow chart of a method for obtaining the intake quantity of a target substance provided in still another embodiment of the present invention. As shown in FIG. 3-2, the method for obtaining the intake quantity of the target substance may include:

Step 310: obtaining a second consumption quantity of the carrier in a valid time period during a single intake process within a statistical period.

In this embodiment, the electronic device is an electronic cigarette, the carrier is a liquid smoke, and the target substance is nicotine, but the present embodiment is not limited.

The valid time period during ingestion refers to the time during which the user smokes the atomized smoke. Optional, can be determined in the following ways:

First, when the electronic cigarette receives the suction signal, it starts to atomize, and stops atomizing when the suction signal is not detected, and the valid time period during the single intake process is the time when the airflow sensor starts detecting the airflow. When the airflow sensor detects the end of the airflow, the airway where the airflow sensor is located communicates with the cigarette holder of the electronic cigarette.

Secondly, the electronic cigarette atomizes when the cigarette lighter button is turned on, and stops the atomization when the cigarette lighter button is turned off, and the valid time period in the single intake process is the time from the cigarette fighter button is turned on to the time the cigarette lighter button is disconnected.

The user can switch the cigarette lighter button in the on state and the off state by operating the cigarette lighter button, and the user can also send a command for turning on or off the electronic cigarette by using a mobile phone, a tablet, or the like. The connect command is used to trigger the electronic cigarette to turn on the cigarette lighter button, and the disconnect command is used to trigger the electronic cigarette to turn off the cigarette lighter button.

Thirdly, the electronic cigarette does not atomize according to the suction signal, but atomizes when the cigarette lighter button is turned on, and stops the atomization when the cigarette lighter button is turned off, in order to obtain a valid time period more accurately, the valid time period is determined in conjunction with the status of the cigarette lighter button and whether a suction signal is detected.

In this method, the single intake process of the electronic cigarette includes receiving the suction signal, stopping receiving the suction signal after receiving the suction signal, turning on the cigarette lighter button, and turning off after the cigarette lighter button is turned on; the starting time of the single intake process is the earlier time of the suction signal is received and the cigarette lighter button is turned on. The cut-off time of the single intake process is the later time of stopping receiving the cigarette lighter signal and the cigarette lighter button is turned off.

The following three ways to illustrate the third way to determine the valid time period are as follows:
In the first case, the first time is earlier than the second time, the start time of the valid time period is the second time, and the cut-off time of the valid time period is the earlier of the third time and the fourth time. The first time is the time when the suction signal is received, the second time is the time when the cigarette lighter button is turned on, the third time is the time when the cigarette lighter button is turned off, and the fourth time is the time when the suction signal is not received.

Since the first time is earlier than the second time, that is, the user first performs suction at the mouthpiece and then operates the cigarette lighter button to smoke, the second time is the time when the user starts to ingest the target substance, so the second time is determined as the start time of the valid time period, to improve the accuracy of the valid time period.

In addition, the earlier of the third time and the fourth time is the time when the user stops ingesting the target substance, so the earlier one of the third time and the fourth time is determined as the cut-off time of the valid time period to improve the accuracy of the valid time period.

In the second case, the first time is later than the second time, and the time interval between the first time and the second time is less than a preset second threshold, and the start time of the valid time period is the second time, cut-off time of the valid time period is the earlier of the third time and the fourth time.

The first time is the time when the suction signal is received, the second time is the time when the cigarette lighter button is turned on, the third time is the time when the cigarette lighter button is turned off, and the fourth time is the time when the suction signal is not received.

The second time is earlier than the first time, that is, the user first operates the cigarette lighter button to light a cigarette, and then smokes at the mouthpiece. Since the time interval between the first time and the second time is less than the preset second threshold, the liquid smoke is atomized into smoke in the time interval and has not evaporated into the air, but during the time interval after the first time, the generated smoke is taken up by the user, so here the start time of the valid time period is the second time.

In the third case, when the first time is later than the second time, and the time interval between the first time and the second time is greater than the threshold, the start time of the valid time period is the first time, and the cut-off time of the valid time period is the earlier of the third time and the fourth time.

The second time is earlier than the first time, that is, the user first operates the cigarette lighter button to light a cigarette, and then smokes at the mouthpiece. Since the time interval between the first time and the second time is greater than the second threshold, it indicates that the smoke generated by the electronic cigarette during the time interval is volatilized into the air but is not taken up by the user, so the time interval cannot be counted into the valid time period. The first time is determined as the start time of the valid time period.

In one example, the following two ways can be used to determine the second consumption quantity of the liquid smoke during a valid time period in a single intake process.

Firstly, the electronic device acquires the third liquid level detected by the liquid level detector at the start time of the valid time period, and acquires the fourth liquid level detected by the liquid level detector at the cut-off time of the valid time period, determines the second consumption quantity of the liquid smoke during the valid time period according to the third liquid level and the fourth liquid level. Wherein, determining the second consumption quantity of the liquid smoke in the valid time period according to the third liquid level and the fourth liquid level can refer to that determining the first consumption quantity of the liquid smoke in the valid time period according to the first liquid level and the second liquid level in the application, and it is not repeated here.

Secondly, in the case that the electronic device is an electronic cigarette, the working mode of the electronic cigarette in each valid time period within the statistical period is acquired; and each unit in each valid time period is calculated according to the working mode of each valid time period. The third consumption quantity of the liquid smoke in the time period; calculating the sum of the third consumption quantity of the liquid smoke in each unit time period in each valid time period, and obtaining the second consumption quantity of the liquid smoke in each valid time period.

The working mode of the electronic cigarette can be, but is not limited to, a temperature control (TC) mode, a variable waits (Variable Walts, VW) mode, and a bypass (Bypass) mode.

The following two cases are explained separately:
The first case: if the electronic cigarette is in the temperature control mode during the valid time period, the third consumption quantity of the liquid smoke in the unit time period is calculated according to the temperature of the atomizing assembly in the unit time period every unit time period.

The temperature of the atomizing assembly may be the temperature at the beginning of the unit time period, the temperature at the end of the unit time period, the average temperature of the temperature at the beginning and the temperature at the end, and the like.

Specifically, in the temperature control mode, at the same temperature, the time required for consuming each 10 ml of liquid smoke is the same, so the time of smoking required to consume 10 ml of liquid smoke at the temperature of the corresponding atomizing assembly can be obtained according to the experimental data. The duration, for example, it takes $3 \times 240$ seconds to consume 10 ml of liquid smoke at 450 degrees Fahrenheit. If the user sucks for 0.1 second at 450 degrees Fahrenheit, the user's consumption of liquid smoke is: $(0.1 \times 10)/(3 \times 240) = 0.0013888889$ ml.

In an embodiment of the present invention, since the liquid smoke is not atomized when smoking below at 150 degrees or 300 degrees Fahrenheit, the third consumption quantity of the liquid smoke is zero.

The second case: if the electronic cigarette is in the variable walts mode or the bypass mode during the valid time period, the third consumption of the liquid smoke in the unit time period is calculated according to the output power of the electronic cigarette in the unit time period every unit time period.

The output power of the electronic cigarette may be the power at the beginning of the unit time period, or the power at the end of the unit time period, or the average value of the power at the beginning and the power at the end.

Specifically, since the generated Joule heat is the same when the electronic cigarette consumes 1 ml of the smoke oil in the variable walts mode or the bypass mode. Therefore, the heat required to consume 1 ml of the liquid smoke can be obtained, according to the experimental data. For example, the heat required to consume 1 ml of the liquid smoke is 2376 joules, and then calculate the heat generated by the e-cigarette according to the power of the e-cigarette and the length of time it works. After calculating the heat, the liquid smoke consumed can be calculated with reference to 2376 joules. For example, if the user sucks for 0.1 second when the power of the electronic cigarette is 20 watts, then the liquid smoke consumed is $(20 \times 0.1)/2376 = 0.00084175$ ml.

Thirdly, in the case that the electronic device is an electronic cigarette, the working mode of the electronic cigarette in each valid time period of the statistical period is obtained; and according to the working mode of each valid time period, the second consumption of the liquid smoke in each valid time period is calculated..

The following two cases are explained separately:

The first case: if the electronic cigarette is in the temperature control mode during the valid time period, the average temperature of the atomizing assembly in the valid time period is obtained, and the second consumption of the liquid smoke in the valid time period is calculated according to the average temperature of the atomizing assembly in the valid time period.

The second case: if the electronic cigarette is in the variable walts mode or the bypass mode during the valid time period, obtaining the average output power of the atomizing assembly in the valid time period, and calculating the second consumption quantity of liquid smoke in the valid time period according to the average output power of the electronic cigarette in the valid time period.

In step 320, obtaining the intake quantity of the target substance within the statistical period, according to the second consumption quantity of the carrier in the valid time period in the single intake process and content of the instruction substance in the carrier.

This step can be implemented in the following two ways:

Firstly, obtaining the second consumption quantity of the carrier in each valid time period within the statistical period, and accumulating the second consumption quantity of the carrier in each valid time period to obtain an accumulated result, and multiplies the accumulated result by the content of the instruction substance in the carrier, to obtaining the intake quantity of the target substance during the statistical period.

Secondly, the second consumption quantity of the carrier in each valid time period within the statistical period is obtained, and the intake quantity of the target substance in each valid time period is calculated according to the second consumption quantity of the carrier in each valid time period, and the intake quantity of the target substance in each valid time period are accumulated to obtain the intake quantity of the target substance within the statistical period.

In summary, the method provided by the embodiment of the present invention obtains the consumption information of the carrier within the statistical period, and the consumption information includes at least one of the first consumption quantity of the carrier within the statistical period, the second consumption quantity of the carrier in a valid time period in a single intake process and a third consumption quantity of the carrier in each unit time period in the valid time period; obtaining the intake quantity of the target substance within the statistical period according to the consumption information and the content of the target substance in the carrier. The user can know the intake quantity of the target substance within the statistical period, thereby helping the user to control the intake quantity of the target substance, and solving problem in the related art that the user ingests too many the target substance in the process of using the electronic cigarette, thereby jeopardizing the health of the body. It plays a reference role in the process of users using e-cigarettes to quit smoking, which can help users to stop smoking actively.

Please refer to FIG. 4, which shows a flow chart of a method for obtaining an intake quantity of a target substance provided in still another embodiment of the present invention. As shown in FIG. 4, the method for obtaining the intake quantity of the target substance may include:

Step 410: obtaining a third consumption quantity of a carrier in each unit time period in the valid time period in the single intake process within a statistical period.

In this embodiment, the electronic device is an electronic cigarette, the carrier is a liquid smoke, and the target substance is nicotine, but the present embodiment is not limited.

For the implementation of obtaining the third consumption quantity of the carrier in each unit time period in the valid time period in the single intake process, reference may be made to the implementation manner in the previous embodiment, and details are not described herein again.

Step 420, obtaining the intake quantity of the target substance within the statistical period according to the third consumption quantity of the carrier in each unit time period and content of the target substance in the carrier.

This step can be achieved in the following ways: Firstly, obtaining the first consumption quantity of the liquid smoke within the statistical period according to the third consumption quantity of the liquid smoke obtained in each unit time period; and obtaining an intake quantity of the nicotine within the statistical period according to the first consumption quantity and the content of nicotine in the liquid smoke .

Specifically, the processor of the electronic cigarette may accumulate the third consumption quantity of the liquid smoke calculated each unit time period in the valid time period in the single intake process, to obtain the second consumption quantity of the liquid smoke in the single intake process, and then accumulate the second consumption quantity of the liquid smoke in each single intake process within the statistical period to obtain the first consumption quantity of the liquid smoke within the statistical period, and then obtain the intake quantity of the nicotine within the statistical period according to the first consumption quantity and content of nicotine of the liquid smoke .

For example, if the user consumes 10 ml of the liquid smoke in the valid time period of the single intake process and consumes 5 ml of the liquid smoke again, then the total consumption quantity of the liquid smoke during the smoking period is 15 ml. By analogy, if the total quantity of liquid smoke consumed during the next smoking period is also 15 ml, and only there are two intake process during the statistical period, the total consumption quantity of the liquid smoke corresponding to the statistical period (ie, the first consumption quantity) is 30 ml. Assuming that the content of nicotine in the liquid smoke is 0.2 mg/ml, the intake quantity of the nicotine within the statistical period is 6 mg.

Secondly, at the end of each unit time period, obtaining the third consumption quantity of the liquid smoke in each unit time period; obtaining the first consumption quantity of the liquid smoke recorded within the statistical period, and accumulating the third consumption quantity and the first consumption quantity, the accumulated result is recorded as the first consumption quantity of the liquid smoke within the statistical period, to update the first consumption quantity of the liquid smoke within the statistical period; and then according to the updated first consumption quantity and the content of nicotine in the liquid smoke updates the intake quantity of nicotine during the statistical period.

Thirdly, for the third consumption of the liquid smoke in each unit time period acquired within the statistical period, calculating the intake quantity of nicotine per unit time period according to the third consumption quantity of the liquid smoke in each unit time period and the content of nicotine in the liquid smoke; calculating the intake quantity of nicotine during the statistical period according to the calculated intake quantity of nicotine per unit time period.

Specifically, calculating the intake quantity of nicotine per unit time period according to the third consumption quantity of the liquid smoke in each unit time period and the content of nicotine in the liquid smoke. Then, the nicotine intake in each unit time period is accumulated in real time to obtain the total nicotine intake during a single intake process, and then accumulating the total nicotine intake during each intake process in the statistical period, to obtain the nicotine intake during the statistical period.

The fourth is to record the intake quantity of nicotine within the statistical period in real time; over time, obtaining the intake quantity of nicotine per unit time period according to the third consumption of liquid smoke per unit time period in the valid time period and the content of nicotine in the liquid smoke; the intake quantity of nicotine per unit time period and the recorded intake quantity of nicotine are added to obtain the new intake quantity of nicotine during the statistical period.

In summary, the method provided by the embodiment of the present invention obtains the consumption information of the carrier within the statistical period, and the consumption information includes at least one of the first consumption quantity of the carrier within the statistical period, the second consumption quantity of the carrier in a valid time period in a single intake process and a third consumption quantity of the carrier in each unit time period in the valid time period; obtaining the intake quantity of the target substance within the statistical period according to the consumption information and the content of the target substance in the carrier. The user can know the intake quantity of the target substance within the statistical period, thereby helping the user to control the intake quantity of the target substance, and solving problem in the related art that the user ingests too many the target substance in the process of using the electronic cigarette, thereby jeopardizing the health of the body. It plays a reference role in the process of users using e-cigarettes to quit smoking, which can help users to stop smoking actively.

In an optional example of any of the above embodiments, after the intake quantity of the target substance within the statistical period is obtained, the related prompt may be performed according to the intake quantity of the target substance, or predetermined operation may be executed according to the intake quantity of the target substance, to help the user control the intake quantity of the target substance. Please refer to the following for details:

The following are several ways to achieve the relevant prompts based on the intake quantity of the target substance:

In one example, the method of displaying the intake quantity of the target substance may further include: displaying a curve of the total quantity of the target substance intake during each intake process within the statistical period.

In one example, after the intake quantity of the target substance within the statistical period is obtained, the intake quantity of the target substance within the statistical period is displayed.

Specifically, for example, but not limited to, the display screen is used to display the total intake quantity of the target substance within the statistical period, and may be, but not limited to, displayed by other means, such as display by voice broadcast.

In a possible implementation manner, the intake quantity of the target substance is correspondingly provided with at least one preset pattern, and the intake quantity of the target substance may be determined according to the actual intake quantity of the target substance within the statistical period, showing the preset pattern. For example, when the intake quantity of the target substance is between 0 mg and 5 mg, the corresponding preset pattern is a smiley face; when the intake quantity of the target substance is between 5 mg and 10 mg, the corresponding preset pattern is a serious face, and the like.

It should be noted that when the user sets a different statistical period, the corresponding relationship between the intake quantity of the target substance and the preset pattern also changes. For example, when the statistical period is 1 day, when the intake quantity of the target substance is 5 mg - 10 mg, it is a serious face; and when the statistical period is one month, the intake quantity of the target substance is 5 mg - 10 mg, it is a smiling face.

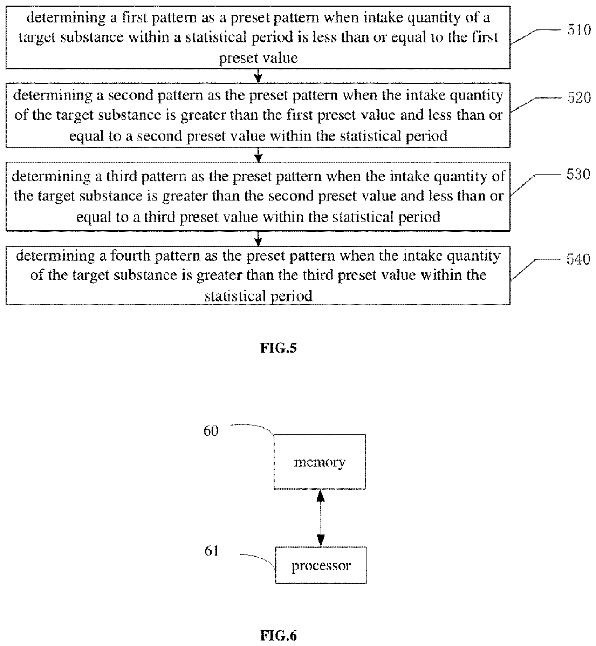

Wherein, determining a preset pattern corresponding to the intake quantity of the target substance within the statistical period; and indicating the intake quantity of the target substance within the statistical period by displaying the preset pattern. This can be achieved by several steps as shown in FIG. 5:

Step 510: determining a first pattern as a preset pattern when intake quantity of a target substance within a statistical period is less than or equal to the first preset value.

In an embodiment, the first pattern may be, for example, but not limited to, a laughing expression.

Step 520: determining a second pattern as the preset pattern when the intake quantity of the target substance is greater than the first preset value and less than or equal to a second preset value within the statistical period.

In an embodiment, the second pattern may be, for example, but not limited to, a smile expression.

Step 530: determining a third pattern as the preset pattern when the intake quantity of the target substance is greater than the second preset value and less than or equal to a third preset value within the statistical period.

In an embodiment, the third pattern may be, for example, but not limited to, a serious expression.

Step 540: determining a fourth pattern as the preset pattern when the intake quantity of the target substance is greater than the third preset value within the statistical period.

In an embodiment, the fourth pattern may be, for example but not limited to, a crying expression.

The first preset value is smaller than the second preset value, and the second preset value is smaller than the third preset value.

Alternatively, laughing expressions, smiling expressions, serious expressions, and showing crying expressions can also be four other patterns that express appreciation and criticism. For example, a first number of flower patterns, a second number of flower patterns, a third number of flower patterns, a fourth number of flower patterns, and the like. The first number is greater than the second number, the second number is greater than the third number, and the third number is greater than the fourth number, which is not limited by the embodiment of the present invention.

By displaying the corresponding preset pattern according to the level of the intake quantity of the target substance within the statistical period, the user can more intuitively obtain the level of the intake quantity of the target substance within the statistical period through the preset group, which can help the user to control The intake quantity of the target substance. For example, in the case where the electronic device is an electronic cigarette and the carrier is a liquid smoke, it can help the user to play a reference role in the smoking cessation process, and help the user to better control the smoking cessation.

In this embodiment, by displaying the intake quantity of the target substance, the user can directly know the intake quantity of the target substance within the statistical period, thereby helping the user to control the intake quantity of the target substance. For example, in the case where the electronic device is an electronic cigarette and the carrier is a liquid smoke, by displaying the intake quantity of nicotine within the statistical period, the user can be helped to play a reference role in the smoking cessation process, thereby helping the user to better control the smoking cessation.

In an example, after obtaining the intake quantity of the target substance within the statistical period, obtaining the first limit intake quantity of the target substance corresponding to the statistical period; calculating the difference between the first limit intake quantity and the intake quantity of target substance within the statistical period, displaying the prompt information according to the difference. Wherein, the prompt information may be used to indicate the remaining ingestible quantity (ie, the difference) of the target substance within the statistical period, and the prompt information may also be used to indicate the remaining smokable mouth number of the electronic cigarette within the statistical period.

Optionally, the remaining smokable mouth number of the electronic cigarette within the statistical period can be obtained by: calculating the average quantity of the target substance ingested by the user according to the user historical suction data; and remaining the target substance according to the statistical period The ingestible quantity and the average quantity of the target substance ingested by the user are counted, and the remaining smokable mouth of the electronic cigarette within the statistical period is calculated.

For example, for example, three mouth number have been sucked by an electronic cigarette, and the average quantity of the target substance per mouth is a mg. If the remaining ingestible quantity of the target substance within the statistical period is b mg, the quotient of b and a is calculated to obtain the remaining smokable number of the electronic cigarette within the statistical period.

The following describes several implementations for controlling the electronic device to perform preset operations based on the intake quantity of the target substance within the statistical period:

In an example, after obtaining the intake quantity of the target substance within the statistical period, detecting whether the intake quantity of the target substance reaches a first threshold corresponding to the statistical period; and the intake quantity of the target substance reaches a first threshold, the output power of the atomizing assembly is lowered, and/or a prompt information for prompting the user that the intake quantity of the target substance is about to exceed the standard is displayed. A solution to control intake while ensuring suction is achieved.

The first threshold may be set by the system developer, or may be customized by the user, or may be determined by the electronic device according to the first limit intake quantity corresponding to the statistical period. For example, the first limit intake quantity corresponding to the statistical period is multiplied by to a predetermined ratio to obtain the first threshold; for example, the first threshold is obtained by subtracting the predetermined value from the first limit intake quantity corresponding to the statistical period.

In an example, after obtaining the intake quantity of the target substance within the statistical period, obtaining the first limit intake quantity of the target substance corresponding to the statistical period, and when the intake quantity of the target substance within the statistical period reaches the first limit intake quantity of the target substance corresponding to the statistical period, the prompt information is displayed, and the prompt information is used to indicate that the intake quantity of the target substance within the statistical period has exceeded the standard.

The manner of displaying the prompt information may be any one of a text prompt, a voice prompt, a buzzer alarm, and the like.

The first limit intake quantity of the target substance corresponding to each statistical period may be set by the system developer or may be customized by the user.

Optionally, when the acquired intake quantity of the target substance of the statistical period reaches the first limit intake quantity of the target substance corresponding to the statistical period, the atomizing assembly is controlled to stop working during the statistical period.

Optionally, a ratio of the intake quantity of the target substance to the first limit intake quantity is calculated, and the ratio is displayed. The way to display the ratio can be any one of a text prompt, a voice prompt, and the like.

Optionally, calculating a ratio of the intake quantity of the target substance to the first limit intake quantity, the ratio is displayed by a progress bar.

In one example, at the end of the statistical period, the limit intake quantity for the next statistical period is determined based on the intake quantity of the target substance within the statistical period.

The specific implementation may be: if the intake quantity of the target substance within the statistical period is lower than the first limit intake quantity corresponding to the statistical period, determining a second limit intake quantity for the next statistical period, the second limit intake quantity is lower than the first limit intake quantity.

The method for obtaining the second limit intake quantity may be: reducing the first limit intake quantity by a predetermined value to obtain the second limit intake quantity, or multiplying the first limit intake quantity by a predetermined ratio to obtain the second limit intake quantity, or determining the intake quantity of the target substance within the statistical period as the second limit intake quantity, which is not specifically limited in this embodiment.

For example, if at the end of a statistical period, the intake quantity of the target substance within the statistical period is 10 mg, and the first limit intake quantity corresponding to the statistical period is 11 mg, the second limit intake quantity of the next statistical period can be determined is 9 mg.

Of course, in actual implementation, the limit intake quantity of the next statistical period can also be determined according to the intake quantity of the previous n statistical period, and will not be described here, and n is a positive integer.

By determining the intake quantity of the next statistical period based on the actual intake quantity before, it is possible to adjust the limit intake quantity according to the user's design intake quantity, thereby assisting the user to quit smoking.

In addition, before performing any of the steps of any of the above embodiments, the electronic device receives an operation instruction input by the user through the input unit, and determines whether to prompt the function of intake quantity of the target substance according to the operation instruction; and the prompt function is enabled. In this case, the steps in any of the above embodiments can be performed.

The input unit may include a button, a touch panel, a voice module, and the like, and the user may also open a prompt function of the intake quantity of the target substance through a terminal device that establishes a communication connection with the electronic cigarette.

In other alternative examples of any of the above embodiments, the prompt function of the intake quantity of the target substance may be turned on by default. Specifically, the user can input a corresponding instruction such as an operation instruction or a mode selection instruction for turning on the intake quantity of the target substance by using an input device such as a button, a touch screen, or a microphone.

In an optional example of any of the above embodiments, the unit time period is a value stored by default in the electronic device, and may be, but is not limited to, a value input by the user through an input device such as a button, a touch screen, or a microphone.

Specifically, the unit time period may be a fixed value, for example, the consumption information of the carrier is collected once in 0.1 seconds; and may also be changed over time, for example, obtaining the consumption information of the carrier every 2 seconds, 1.5 seconds, 1 second, 0.5 seconds, 0.1 seconds.

For example, obtaining the consumption information of the carrier 2 seconds after the first time to obtain the consumption information of the carrier, obtaining the consumption information of the carrier again 1.5 seconds after the second time to obtain the consumption information of the carrier, and obtaining the consumption information of the carrier 1 second after the three times to obtain the consumption information of the carrier, obtaining the consumption information of the carrier again 0.5 seconds after the fourth time to obtain the consumption information of the carrier, obtaining the consumption information of the carrier again 0.1 seconds after the fifth time to obtain the consumption information of the carrier, and then obtain the consumption information of the carrier every 0.1 seconds.

FIG. 6 is a schematic diagram of a module of an electronic device provided by the present invention. As shown in FIG. 6, the electronic device includes a memory 60 and a processor 61.

The memory 60 is configured to storing executable program code; and the processor 61 is configured to call the executable program code in the memory to perform the above-described method of obtaining the intake quantity of the target substance.

In an embodiment of the invention, the electronic device further includes an input module (not shown) for receiving an instruction input by the user.

In an embodiment of the invention, the electronic device further includes a display module (not shown) for displaying the intake quantity of the target substance within the statistical period.

In an embodiment of the invention, the electronic device further includes an atomizing assembly (not shown) for atomizing the carrier for the user to smoke.

The above is only a preferred embodiment of the present invention, and is not intended to limit the scope of the present invention. Although the present invention has been disclosed in the above preferred embodiments, it is not intended to limit the present invention. The skilled person can make some modifications or modifications to the equivalent embodiments by using the technical content disclosed above without departing from the technical scope of the present invention, but without departing from the technical scope of the present invention, according to the present invention. Technical Substantials Any simple modifications, equivalent changes and modifications made to the above embodiments are still within the scope of the technical solutions of the present invention.

A person skilled in the art may understand that all or part of the steps of implementing the above embodiments may be completed by hardware, or may be instructed by a program to execute related hardware, and the program may be stored in a computer readable storage medium. The storage medium mentioned may be a read only memory, a magnetic disk or an optical disk or the like.

**Claims**

1. A method for obtaining an intake quantity of a target substance, **characterized in that**, the method comprises:

obtaining consumption information of a carrier within a statistical period, the consumption information comprising at least one of a first consumption quantity of the carrier within the statistical period, a second consumption quantity of the carrier in a valid time period in a single intake process, and a third consumption quantity of the carrier in each unit time period of the valid time period;
obtaining the intake quantity of the target substance within the statistical period according to the consumption information and content of the target substance in the carrier.

2. The method according to claim 1, **characterized in that**, the obtaining the consumption information of a carrier within a statistical period comprises:

    using a liquid level detector to detect a first change quantity of liquid level within the statistical period, and determining the first consumption quantity according to the first change quantity; or,
    using the liquid level detector to detect a second change quantity of the liquid level in the valid time period, and determining the second consumption quantity according to the second change quantity.

3. The method according to claim 1, **characterized in that**, the obtaining the consumption information of a carrier within a statistical period comprises:

    obtaining a working mode of an electronic cigarette in each valid period of time within the statistical period;
    obtaining the consumption information of the carrier within the statistical period according to the working mode of each valid time period.

4. The method according to claim 1, **characterized in that**, the obtaining the consumption information of the carrier within the statistical period according to the working mode of each valid time period comprises:

    if the electronic cigarette is in a temperature control mode during the valid time period, calculating the third consumption quantity of the carrier in the unit time period according to the temperature of an atomizing assembly in the unit time period every unit time period; or,
    if the electronic cigarette is in the temperature control mode during the valid time period, obtaining an average temperature of the atomizing assembly in the valid time period; and calculating the second consumption of the carrier during the valid time period according to the average temperature of the atomizing assembly in the valid time period.

5. The method according to claim 1, **characterized in that**, the obtaining the consumption information of the carrier within the statistical period according to the working mode of each valid time period comprises:

    if the electronic cigarette is in a variable walts mode or a bypass mode during the valid time period, calculating the third consumption quantity of the carrier in the unit time period according to output power of the electronic cigarette in the unit time period every unit time period; or,
    obtaining an average output power of the electronic cigarette in the valid time period if the electronic cigarette is in the variable walts mode or the bypass mode during the valid time period; and calculating the second consumption quantity of the carrier during the valid time period according to the average output power of the electronic cigarette in the valid time period.

6. The method according to any one of claims I to 5, **characterized in that**, after obtaining the intake quantity of the target substance within the statistical period, the method further comprises:
controlling the electronic device to perform a preset operation according to the intake quantity.

7. The method of claim 6, **characterized in that**, controlling the electronic device to perform a preset operation according to the intake quantity comprises:

    detecting whether the intake quantity reaches a first threshold corresponding to the statistical period;
    when the intake quantity reaches the first threshold, reducing the output power of the atomizing assembly, and/or presenting prompt information for prompting an user that the intake quantity is about to exceed the limit.

8. The method of claim 6, **characterized in that**, controlling the electronic device to perform a preset operation according to the intake quantity comprises:
determining a limit intake quantity of a next statistical period according to the intake quantity of the target substance during the statistical period, at the end of the statistical period.

9. The method according to any one of claims 1 to 5, **characterized in that**, after obtaining the intake quantity of the target substance within the statistical period, the method further comprises:
displaying prompt information according to the intake quantity, and the prompt information is used to prompt the intake quantity of the target substance.

10. The method of claim 9, **characterized in that**, displaying prompt information according to the intake quantity comprises:

    determining a preset pattern corresponding to the intake quantity;
    prompting the intake quantity by displaying the preset pattern; or
displaying the intake quantity of the target substance within the statistical period.

11. The method according to any one of claims 1 to 5, **characterized in that**, after obtaining the intake quantity of the target substance within the statistical period, the method further comprises:

    obtaining a first limit intake quantity of the target substance corresponding to the statistical period;
    calculating a ratio of the intake quantity to the first limit intake quantity;
    displaying the ratio, or displaying the ratio by displaying a progress bar.

12. The method according to any one of claims 1 to 5, **characterized in that**, the valid time period is a time when an airflow sensor starts detecting the airflow to a time when the airflow sensor detects the end of the airflow.

13. The method according to any one of claims 1 to 5, **characterized in that**, the valid time period is a time when a cigarette lighter button is turned on to a time when the cigarette lighter button is turned off.

14. A method according to any one of claims 1 to 5, **characterized in that**,
if a first time is earlier than a second time, start time of the valid time period is the second time; and/or,
if the first time is later than the second time, and time interval between the first time and the second time is less than a second threshold, the start time of the valid time period is the second time; and/or,
if the first time is later than the second time, and the time interval between the first time and the second time is greater than the second threshold, the start time of the valid time period is the first time;
and/or, the cut-off time of the valid time period is an earlier one of the third time and the fourth time;
wherein the first time is the time when a suction signal is received, the second time is the time when the cigarette lighter button is turned on, and the third time is the time when a cigarette lighter button is turned off, the fourth time is the time when the suction signal is not received.

15. An electronic device, **characterized in that**, the device comprises: a memory and a processor;
the memory is configured to storing executable program code;
the processor is configured to invoke the executable program code in the memory to execute the method of obtaining an intake quantity of target substance according to any one of claims **1-14.**

obtaining consumption information of a carrier within a statistical period, the consumption information includes at least one of a first consumption quantity of the carrier within the statistical period, a second consumption quantity of the carrier in the valid time period in a single intake process, and a third consumption of the carrier per unit time period within the statistical period — 110

obtaining an intake quantity of the target substance within the statistical period according to the consumption information of the carrier and content of the target substance in the carrier — 120

FIG.1

obtaining a first consumption quantity of a carrier within a statistical period, in the statistical period — 210

obtaining an intake quantity of the target substance within the statistical period according to the first consumption quantity of the carrier and the content of the target substance in the carrier — 220

FIG.2

**FIG.3-1**

| obtaining a second consumption quantity of the carrier in a valid time period during a single intake process within a statistical period | 310 |
|---|---|
| obtaining the intake quantity of the target substance within the statistical period, according to the second consumption quantity of the carrier in the valid time period in the single intake process and content of the instruction substance in the carrier | 320 |

**FIG.3-2**

| obtaining a third consumption quantity of a carrier in each unit time period in the valid time period in the single intake process within a statistical period | 410 |
|---|---|
| obtaining the intake quantity of the target substance within the statistical period according to the third consumption quantity of the carrier in each unit time period and content of the target substance in the carrier | 420 |

**FIG.4**

determining a first pattern as a preset pattern when intake quantity of a target substance within a statistical period is less than or equal to the first preset value ⌐ 510

determining a second pattern as the preset pattern when the intake quantity of the target substance is greater than the first preset value and less than or equal to a second preset value within the statistical period ⌐ 520

determining a third pattern as the preset pattern when the intake quantity of the target substance is greater than the second preset value and less than or equal to a third preset value within the statistical period ⌐ 530

determining a fourth pattern as the preset pattern when the intake quantity of the target substance is greater than the third preset value within the statistical period ⌐ 540

**FIG.5**

60

memory

61

processor

**FIG.6**

| | **INTERNATIONAL SEARCH REPORT** | International application No. |
| --- | --- | --- |
| | | PCT/CN2017/118838 |

**A. CLASSIFICATION OF SUBJECT MATTER**

A24F 47/00 (2006.01) i

According to International Patent Classification (IPC) or to both national classification and IPC

**B. FIELDS SEARCHED**

Minimum documentation searched (classification system followed by classification symbols)

A24F 47 A61M 5

Documentation searched other than minimum documentation to the extent that such documents are included in the fields searched

Electronic data base consulted during the international search (name of data base and, where practicable, search terms used)

CNABS; CNTXT; VEN; USTXT: 吸入量, 电子烟, 摄入量, 药, 常州市派腾电子, 周期, 尼古丁, 摄取量, 吸烟量, 摄取量, 吸取量, 液位, 戒烟, control, aerosol, smok+, electronic cigarette, vaper, nicotine

**C. DOCUMENTS CONSIDERED TO BE RELEVANT**

| Category* | Citation of document, with indication, where appropriate, of the relevant passages | Relevant to claim No. |
| --- | --- | --- |
| X | CN 106535673 A (SMOKEWATCHERS S A S) 22 March 2017 (22.03.2017), description, paragraphs [0047], [0051], [0052], [0054] and [0065], and figures 1-6 | 1-15 |
| A | CN 205321198 U (YANG, Zhilin) 22 June 2016 (22.06.2016), entire document | 1-15 |
| A | US 7164993 B2 (PLOWSHARE TECHNOLOGIES INC.) 16 January 2007 (16.01.2007), entire document | 1-15 |
| A | CN 1761412 A (BLOENDAL STEINGRIMUR TH) 19 April 2006 (19.04.2006), entire document | 1-15 |

☐ Further documents are listed in the continuation of Box C.   ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 10 May 2018 | 24 May 2018 |

| Name and mailing address of the ISA | Authorized officer |
| --- | --- |
| State Intellectual Property Office of the P. R. China No. 6, Xitucheng Road, Jimenqiao Haidian District, Beijing 100088, China Facsimile No. (86-10) 62019451 | GAO, Shizhi Telephone No. (86-512) 88997395 |

Form PCT/ISA/210 (second sheet) (July 2009)

## INTERNATIONAL SEARCH REPORT
### Information on patent family members

International application No.

PCT/CN2017/118838

| Patent Documents referred in the Report | Publication Date | Patent Family | Publication Date |
|---|---|---|---|
| CN 106535673 A | 22 March 2017 | US 2016278435 A1 | 29 September 2016 |
| | | PH 12016500815 A1 | 13 June 2016 |
| | | CA 2928868 A1 | 07 May 2015 |
| | | WO 2015063126 A1 | 07 May 2015 |
| | | EP 3062643 A1 | 07 September 2016 |
| | | HK 1228684 A0 | 10 November 2017 |
| CN 205321198 U | 22 June 2016 | None | |
| US 7164993 B2 | 16 January 2007 | US 2004030508 A1 | 12 February 2004 |
| CN 1761412 A | 19 April 2006 | WO 2004075671 A8 | 29 September 2005 |
| | | EP 1596676 A1 | 23 November 2005 |
| | | JP 2006518619 A | 17 August 2006 |
| | | US 2006200322 A1 | 07 September 2006 |
| | | WO 2004075671 A1 | 10 September 2004 |

Form PCT/ISA /210 (patent family annex) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 200910818181